(19) Europäisches Patentamt
European Patent Office
Office européen des brevets

(11) Veröffentlichungsnummer: **0 008 118**
**B1**

(12) # EUROPÄISCHE PATENTSCHRIFT

(45) Veröffentlichungstag der Patentschrift: **07.01.81**

(51) Int. Cl.³: **C 09 B 11/24,** C 07 D 307/88

(21) Anmeldenummer: **79102873.1**

(22) Anmeldetag: **09.08.79**

(54) **Verfahren zur Herstellung von 3,3-Bis-(4-dimethylaminophenyl)-6-dimethylaminophthalid.**

(30) Priorität: **12.08.78 DE 2835450**

(43) Veröffentlichungstag der Anmeldung: **20.02.80 Patentblatt 80/4**

(45) Bekanntmachung des Hinweises auf die Patenterteilung: **07.01.81 Patentblatt 81/1**

(84) Benannte Vertragsstaaten: **BE CH DE FR GB IT**

(56) Entgegenhaltungen:
**DE-A-2 018 168**
**DE-A-2 016 375**
**US-A-3 642 514**

(73) Patentinhaber: **BASF Aktiengesellschaft, Carl-Bosch-Strasse 38, D-6700 Ludwigshafen (DE)**

(72) Erfinder: **Seibert, Walter, Dr., Leuschnerstrasse 46, D-6700 Ludwigshafen (DE)**

**EP 0 008 118 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

ACTORUM AG.

# Verfahren zur Herstellung von 3,3-Bis-(4-dimethylaminophenyl)-6-dimethylaminophthalid

Die Erfindung betrifft ein Verfahren zur Herstellung von 3,3-Bis-(4-dimethylaminophenyl)-6-dimethylaminophthalid (=Kristallviolettlacton) durch Oxidation von 2-(4,4'-Bis-dimethylaminobenzhydryl)-5-dimethylaminobenzoesäure.

3,3-Bis-(4-dimethylaminophenyl)-6-dimethylaminophthalid, im folgenden als Kristallviolettlacton oder KVL bezeichnet, hat als Farbbildner für druckempfindliche Kopier- und Durchschreibesysteme eine grosse Bedeutung.

Aus der Patentliteratur sind bereits verschiedene Verfahren zur Oxidation von 2-(4,4'-Bis-dialkylaminobenzhydryl)-5-dialkylaminobenzoesäuren, insbesondere von 2-(4,4'-Bis-dimethylaminobenzhydryl)-5-dimethylaminobenzoesäure in saurer und in alkalischer Lösung bekannt (DE-OS 2557687; DE-OS 2156662; DE-OS 1962881; DE-OS 2143021; US-PS 3987062, 4076728 und 3185709; JA-OS 124931/1975 referriert in C.A. 84 (1976) 46059p).

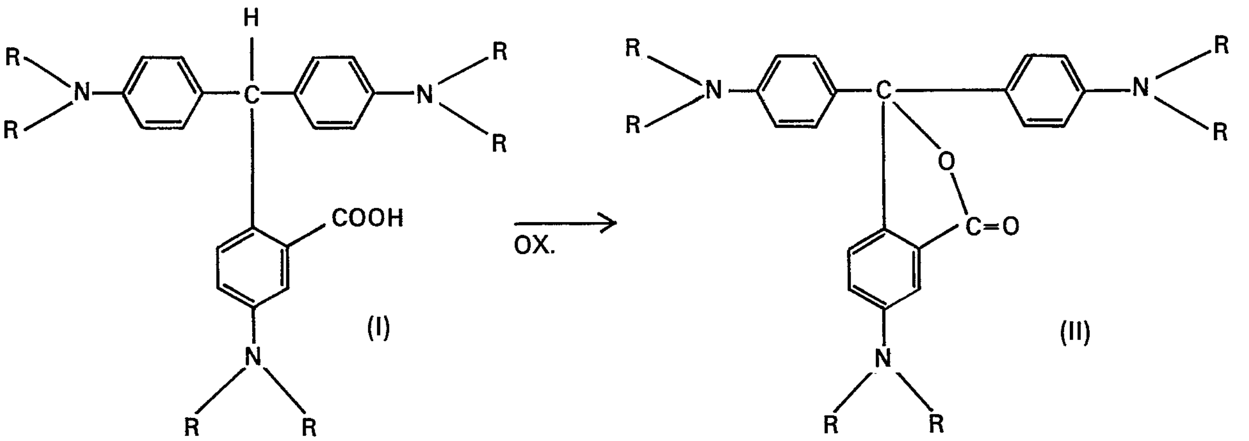


(I) (II)

R = Alkyl, insbesondere - $CH_3$

Die Oxidation in alkalischer Lösung bietet sich vor allem dann an, wenn die Säure nach der Synthese oder Reinigung in Form einer verdünnten wässrigen Lösung vorliegt. In vielen Fällen wird zur Verbesserung der Teilchenform des Reaktionsproduktes und/oder zur Verbesserung der Ausbeute die Oxidation in Gegenwart von organischen Lösungsmiteln durchgeführt (DE-OS 2156662; US-PS 3987062; JA-OS 124931/1975).

Die Oxidationsverfahren des Standes der Technik sind hinsichtlich der Ausbeute an dem Kristallviolettlacton nicht optimal. Verfahren, bei denen in Wasser in Gegenwart von organischen Lösungsmitteln gearbeitet wird, haben den Nachteil, dass aus ökologischen Gründen die in der Mutterlauge enthaltenen Lösungsmittel entweder wiedergewonnen oder auf andere Weise, z.B. durch biologischen Abbau entfernt werden müssen. Beide Möglichkeiten der Entfernung der Lösungsmittel verursachen hohe Kosten.

Aufgabe der vorliegenden Erfindung war es, ein Oxidationsverfahren für 2-(4,4'-Bis-dimethylaminbenzydryl)-5-dimethylaminobenzoesäure aufzufinden, das in Abwesenheit von organischen Lösungsmitteln das entsprechende Phthalid (II) in hoher Ausbeute und möglichst in verbesserter Reinheit liefert.

Es wurde gefunden, dass man - 3,3-Bis-(4'-dimethylaminophenyl)-6-dimethylaminophthalid - (Kristallviolettlacton) in hoher Ausbeute und guter Reinheit durch Oxidation von 2-(4,4'-Bis-dimethylaminobenzhydryl)-5-dimethylaminobenzoesäure in wässrig-alkalischer Lösung bei Temperaturen zwischen 0 und 100°C und im pH-Bereich zwischen 8 und 12 erhält, wenn man die Oxidation mit wasserlöslichen Salzen des Hexacyanoferrat-III oder in Gegenwart von Hexacyanoferrat-III durchführt.

Nach dem erfindungsgemässen Verfahren erhält man nach dem Umkristallisieren Kristallviolettlacton mit einem Reingehalt von 98 bis 100% in einer Ausbeute von 88 bis 92% der Theorie. Die hohe Ausbeute an Kristallviolettlacton war überraschend, da aus J. Org. Chem. 16 (1951), Seite 1303 bekannt ist, dass tertiäre, Methylgruppen enthaltende aliphatische Amine durch alkalische Kaliumhexacyanoferrat-III-Lösungen entmethyliert werden. Weiterhin ist aus dieser Literatur bekannt, dass Hexacyanoferrat-III in alkalischer Lösung auch durch Dimethylanilin reduziert wird. Die nach dem erfindungsgemässen Verfahren erhaltene Ausbeute an KVL ist wesentlich höher als bei dem in der DE-OS 2143021 beschriebenen Oxidationsverfahren mit Peroxidisulfaten. Aus den Angaben des Beispiels 2 errechnet sich eine Ausbeute von 72% der Theorie an gereinigtem KVL, für das jedoch keine Angaben über die Reinheit gemacht werden.

Das erfindungsgemässe Verfahren wird in der Regel so durchgeführt, dass man die 2-(4,4'-Bis-dimethylaminobenzhydryl)-5-dimethylaminobenzoesäure, im folgenden auch als DABS bezeichnet, nach Zugabe der erforderlichen Menge eines Alkalimetallcarbonates oder Alkalimetallhydroxids in Wasser in der Wärme löst und mit den wasserlöslichen Salzen des Hexacyanoferrat-III die Säure zum Lacton oxidiert.

Das Verhältnis von DABS zu Wasser beträgt im allgemeinen das 5- bis 20fache der Gewichtsmenge an DABS. Die Menge an Alkalimetallcarbonat und/oder Alkalimetallhydroxid beträgt

mindestens 1,2 Äquivalent je Mol DABS, vorteilhafterweise wendet man 4 bis 7 Äquivalent dieser Mittel je Mol DABS an. Als Alkalimetallcarbonat kommt z.B. Kaliumcarbonat und vorzugsweise Natriumcarbonat, als Alkalimetallhydroxid vor allem Kalium- und Natriumhydroxid in Betracht.

Die Menge an wasserlöslichem Salz des Hexacyanoferrat-III beträgt mindestens die stöchiometrisch erforderliche Menge. Vorzugsweise wendet man das 1,0- bis 1,2fache der stöchiometrisch erforderlichen Menge, d.h. 2,0 bis 2,4 Mol Hexacyanoferrat-III je Mol DABS, an.

Als wasserlösliche Salze des Hexacyanoferrat-III kommen vor allem die des Kaliums und Natriums in Betracht. Anstelle des handelsüblichen Kaliumhexacyanoferrat-III können auch Lösungen verwendet werden, die durch Oxidation von Kaliumhexacyanoferrat-II in Gegenwart von Säuren mit Wasserstoffperoxid bei Raumtemperatur erhalten werden.

Die Oxidation der DABS kann bei Temperaturen zwischen 0 und 100 °C erfolgen. Vorteilhafterweise arbeitet man im Bereich zwischen Raumtemperatur (etwa 20 °C) und 100 °C, vorzugsweise zwischen 30 und 100 °C.

Die Oxidation erfolgt rasch und ist im allgemeinen in weniger als 5 Stunden beendet.

Für die Reaktionsgeschwindigkeit und für die Ausbeute ist entscheidend, dass der pH-Wert im Reaktionsgemisch nicht unter 8 sinkt. Gegebenenfalls wird durch Nachgeben von Soda, Kaliumcarbonat und/oder Alkalihydroxidlösungen der pH-Wert oberhalb von 8 gehalten.

Nach der Beendigung der Oxidation erfolgt die Isolierung des Verfahrensproduktes in üblicher Weise. Das rohe Lacton wird aus der warmen bis heissen Lösung abfiltriert und mit Wasser neutral gewaschen. Das erhaltene rohe Lacton wird dann durch Umkristallisieren gereinigt. Gegebenenfalls können dabei noch vorhandene basische Anteile durch Zugeben einer geringen Menge einer organischen Carbonsäure, z.B. Essigsäure, neutralisiert werden. Man erhält so ein etwa reineres KVL. Das umkristallisierte Kristallviolettlacton hat im allgemeinen einen Reingehalt von 98% und darüber.

Das erfindungsgemässe Verfahren kann auch so durchgeführt werden, dass man ein wasserlösliches Salz des Hexacyanoferrat-II, in einer Menge, die mindestens der stöchiometrisch erforderlichen Menge an Hexacyanoferrat-III äquivalent ist, verwendet und mindestens die stöchiometrisch erforderliche Menge eines Oxidationsmittels, das Hexacyanoferrat-II zum Hexacyanoferrat-III oxidieren kann, zugibt.

Man kann aber auch geringere als die stöchiometrisch erforderliche Menge bis katalytische Mengen an Hexacyanoferrat-III und/oder Hexacyanoferrat-II und Oxidationsmittel, die das Hexacyanoferrat-II zu Ferrat-III oxidieren, anwenden.

Unter katalytischer Menge wird im Sinne der vorliegenden Erfindung eine Menge an Hexacyanoferrat-II – und/oder -III von 2 bis 30%, vorzugsweise von 5 bis 20%, der stöchiometrisch benötigten Menge verstanden. Als Oxidationsmittel, die Hexacyanoferrat-II zum -Ferrat-III oxidieren, kommen vor allem Wasserstoffperoxid und Peroxidisulfate wie Kalium-, Natrium- oder Ammoniumperoxidisulfat in Betracht. Wasserstoffperoxid kann in den handelsüblichen Konzentrationen, die zwischen 3 und 90 Gew.% liegen, angewendet werden. Vorzugsweise wird man jedoch Lösungen mit 30 Gew.% und höheren Gehalten verwenden. Von diesen Oxidationsmitteln wird mindestens soviel angewendet, dass im Falle eines Unterschusses an Hexacyanoferrat-III die Summe aus Hexacyanoferrat-III und dem weiteren Oxidationsmittel mindestens der stöchiometrisch erforderlichen Menge entspricht. Für den Fall, dass man mit katalytischen Mengen an Hexacyanoferrat-II und/oder -III arbeitet, wendet man mindestens die stöchiometrisch erforderliche Menge an Oxidationsmittel, bei Wasserstoffperoxid vorzugsweise 1 bis 4 Mol je Mol DABS an. Bei Peroxidisulfaten wendet man vorzugsweise die stöchiometrisch erforderliche Menge bis zum 1,2fachen dieser Menge an (entsprechend 1 bis 1,2 Mol je Mol DABS).

Die Oxidation erfolgt bei diesem Varianten des erfindungsgemässen Verfahrens vorteilhafterweise bei Temperaturen zwischen 70 und 100 °C. Die Reaktion ist bei diesen Temperaturen in der Regel in weniger als 5 Stunden beendet.

Aufarbeitung, Isolierung des Kristallviolettlactons und dessen Reinigung durch Umkristallisieren erfolgen wie oben angegeben.

Das erfindungsgemässe Verfahren soll durch die folgenden Ausführungsbeispiele weiter erläutert werden. Der Gehalt der als Ausgangsstoff verwendeten 2-(4,4′-Bis-dimethylaminobenzhydryl)-5-dimethylaminobenzoesäure wurde durch Titration mit Tetrabutylammoniumhydroxid (potentiometrische Anzeige) bestimmt. Der Gehalt lag bei dem verwendeten Produkt bei 99,1%. Der Reingehalt des Kristallviolettlactons wurde spektroskopisch bei 600 nm bestimmt.

Die im folgenden angegebenen Teile und Prozentangaben beziehen sich auf das Gewicht.

## Beispiel 1

83,4 Teile 2-(4,4′-Bis-dimethylamino-benzhydryl)-5-dimethylaminobenzoesäure (100%ig) werden in 1000 Teilen Wasser nach Zusatz von 50 Teilen kalzinierter Soda unter Erwärmen in Lösung gebracht. Es stellt sich ein pH-Wert von 9,6 bei 60 °C ein. Man lässt bei dieser Temperatur eine Lösung von 135 Teilen Kaliumhexacyanoferrat-III in 300 Teilen Wasser in 40 Minuten zulaufen, wobei der pH-Wert auf etwa 8,8 absinkt. Man erwärmt 5 Minuten auf 80 °C, um eine besser filtrierbare Form des Reaktionsproduktes zu erhalten. Das entstandene kristalline rohe Kristallviolettlacton wird abfiltriert und mit Wasser gewaschen. Zur Reinigung versetzt man das feuchte Rohlacton (138 Teile) mit der gleichen Gewichtsmenge Äthylenglykolmonobutyläther (Butylglykol) erhitzt das Gemisch auf 145 °C, wobei Wasser abdestilliert und das Lacton in Lösung geht. Man

filtriert heiss von geringen Mengen eines anorganischen Rückstandes ab und lässt kristallisieren. Das Kristallisat wird abgesaugt, mit wenig Butylglykol gewaschen und getrocknet. Ausbeute 76,4 Teile KVL mit einem Schmelzpunkt von 179 bis 180 °C und einem Reingehalt von 100%. Dies entspricht einer Ausbeute von 92,0% der Theorie.

Beispiel 2

83,4 Teile 2-(4,4'-Bis-dimethylamino-benzhydryl)-5-dimethylaminobenzoesäure (100%ig) werden in 1000 Teilen Wasser unter Zusatz von 70 Teilen kalzinierter Soda bei 60 °C gelöst. Man lässt unter Rühren auf 30 °C abkühlen, wobei der grösste Teil der Säure als feinkristallines Natriumsalz ausfällt (pH-Wert 10,3). Nach Zusatz von 30 Teilen Kaliumhexacyanoferrat-II lässt man in 25 Minuten eine Lösung von 53 Teilen Natriumpersulfat in 150 Teilen Wasser zulaufen, wobei die Temperatur auf 42 °C ansteigt und der pH-Wert auf 9,6 sinkt. Man erwärmt auf 80 °C, um ein gut filtrierbares Produkt zu erhalten. Isolierung und Reinigung erfolgen wie in Beispiel 1 angegeben. Ausbeute 74,2 Teile KVL mit einem Schmelzpunkt von 178 bis 179,5 °C und einem Reingehalt von 98,9%. Dies entspricht einer Ausbeute von 88,4% der Theorie.

Beispiel 3

83,4 Teile 2-(4,4'-Bis-dimethylamino-benzhydryl)-5-dimethylaminobenzoesäure (100%ig) werden in 1000 Teilen Wasser unter Zusatz von 70 Teilen kalzinierter Soda bei 60 °C gelöst. Man lässt unter Rühren auf 30 °C abkühlen, wobei die Säure grösstenteils als feinkristallines Natriumsalz ausfällt (pH-Wert = 10,0). Dann lässt man in 3 Minuten eine etwa 6 Stunden alte, bei Raumtemperatur hergestellte Mischung aus 173 Teilen Kaliumhexacyanoferrat-II, 200 Teilen 10%iger Schwefelsäure und 125 Teilen 6%igem Wasserstoffperoxid zulaufen. Unter geringem Temperaturanstieg nimmt der pH-Wert auf etwa 9,5 ab. Man erwärmt auf 80 °C, um ein gut filtrierbares Produkt zu erhalten.

Isolierung und Reinigung erfolgen wie im Beispiel 1 angegeben. Ausbeute 77,0 Teile KVL mit einem Schmelzpunkt von 178 bis 180 °C und einem Reingehalt von 98,5%. Dies entspricht einer Ausbeute von 91,4% der Theorie.

Beispiel 4

83,4 Teile 2-(4,4'-Bis-dimethylamino-benzhydryl)-5-dimethylaminobenzoesäure (100%ig) werden in 1000 Teilen Wasser unter Zusatz von 70 Teilen kalzinierter Soda bei erhöhter Temperatur gelöst. Nach Zusatz von 43,25 Teilen Kaliumhexacyanoferrat-II erhitzt man auf 95 °C und lässt in 4 Stunden 40,8 Teile Wasserstoffperoxid (50%ig) zutropfen. Man rührt 20 Minuten nach und saugt das grobkristalline Lacton heiss ab.

Die Reinigung erfolgt entsprechend den Angaben des Beispiels 1. Ausbeute 75,3 Teile KVL mit einem Schmelzpunkt von 176 bis 178 °C und einem Reingehalt von 98,5%. Dies entspricht einer Ausbeute von 89,6% der Theorie.

Beispiel 5

83,4 Teile 2-(4,4'-Bis-dimethylamino-benzhydryl)-5-dimethylaminobenzoesäure (100%ig) werden in 1000 Teilen Wasser unter Zugabe von 0,2 Mol Natriumhydroxid gelöst. In der Lösung wird der pH-Wert durch Zugabe von Natronlauge auf pH = 10 bis 10,5 eingestellt. Die Oxidation erfolgt wie in Beispiel 4 angegeben, jedoch wird der pH-Wert im Reaktionsgemisch durch Zugeben von Natronlauge zwischen 9,5 und 10 gehalten.

Das Verfahrensprodukt wird wie in Beispiel 4 aufgearbeitet und umkristallisiert.

Die Ausbeute und die Reinheit des Produktes entsprechen dem nach Beispiel 4 erhältlichen.

**Patentansprüche**

1. Verfahren zur Herstellung von 3,3-Bis-(4-dimethylaminophenyl)-6-dimethylaminophthalid (Kristallviolettlacton) durch Oxidation von 2-(4,4'-Bis-dimethylaminobenzhydryl)-5-dimethylaminobenzoesäure in wässrig alkalischer Lösung bei Temperaturen zwischen 0 und 100 °C und im pH-Bereich zwischen 8 und 12, dadurch gekennzeichnet, dass man die Oxidation mit wasserlöslichen Salzen des Hexacyanoferrat-III oder in Gegenwart von Hexacyanoferrat-III durchführt.

2. Verfahren gemäss Anspruch 1, dadurch gekennzeichnet, dass man mindestens die stöchiometrisch erforderliche Menge an wasserlöslichen Salzen des Hexacyanoferrat-III anwendet.

3. Verfahren gemäss Anspruch 1, dadurch gekennzeichnet, dass man eine geringere als die stöchiometrisch erforderliche bis katalytische Menge an Hexacyanoferrat-II und/oder -III und Oxidationsmittel anwendet, die Hexacyanoferrat-II zu Hexacyanoferrat-III oxidieren.

4. Verfahren gemäss Anspruch 3, dadurch gekennzeichnet, dass man als Oxidationsmittel Alkalimetallperoxidisulfat, Ammoniumperoxidisulfat, Wasserstoffperoxid oder Gemische davon verwendet.

5. Verfahren gemäss Anspruch 3, dadurch gekennzeichnet, dass man je Mol 2-(4,4'-Bis-dimethylaminobenzhydryl)-5-dimethylaminobenzoesäure 1 bis 4 Mol Wasserstoffperoxid oder 1 bis 1,2 Mol Peroxidisulfat anwendet.

**Claims**

1. A process for the preparation of 3,3-bis-(4-dimethylaminophenyl)-6-dimethylaminophthalide (crystal violet lactone) by oxidizing 2-(4,4'-bis-dimethylaminobenzhydryl)-5-dimethylaminobenzoic acid in aqueous alkaline solution at from 0 to 100 °C and at a pH of from 8 to 12, wherein the oxidation is carried out with a water-soluble ferricyanide salt or in the presence of a ferricyanide.

2. A process as claimed in claim 1, wherein not less than the stoichiometrically required amount of a water-soluble ferricyanide salt is employed.

3. A process as claimed in claim 1, wherein an amount which is less than that stoichiometrically required, and may be merely a catalytic amount, of a ferrocyanide and/or ferricyanide is used together with an oxidizing agent which oxidizes a ferrocyanide to a ferricyanide.

4. A process as claimed in claim 3, wherein an alkali metal peroxydisulfate, ammonium peroxydisulfate, hydrogen peroxide or a mixture of these is used as the oxidizing agent.

5. A process as claimed in claim 3, wherein from 1 to 4 moles of hydrogen peroxide or from 1 to 1.2 moles of peroxydisulfate are used per mole of 2-(4,4'-bis-dimethylaminobenzhydryl)-5-dimethylaminobenzoic acid.

**Revendications**

1. Procédé de préparation du 3,3-bis-(4-diméthylaminophényl)-6-diméthylamino-phtalide (lactone du Violet cristallisé) par oxydation de l'acide 2-(4,4'-bis-diméthylaminobenzhydryl)-5-diméthylamino-benzoïque en solution aqueuse alcaline d'un pH compris entre 8 et 12, à une température comprise entre 0 et 100 °C, caractérisé en ce que l'oxydation est effectuée à l'aide d'un ferricyanure soluble dans l'eau ou en présence d'un ferricyanure.

2. Procédé suivant la revendication 1, caractérisé en ce que le ferricyanure hydrosoluble est mis en œuvre en une proportion correspondant au moins à la proportion stœchiométrique.

3. Procédé suivant la revendication 1, caractérisé en ce que l'on emploie une proportion de ferricyanure et (ou) de ferrocyanure et d'un agent oxydant capable d'oxyder le ferrocyanure en ferricyanure inférieure à la proportion stœchiométrique et pouvant se réduire à une proportion catalytique.

4. Procédé suivant la revendication 3, caractérisé en ce que l'agent oxydant est choisi parmi les peroxy-disulfates de métaux alcalins, le peroxydisulfate d'ammonium et le peroxyde d'hydrogène, ainsi que leurs mélanges.

5. Procédé suivant la revendication 3, caractérisé en ce que l'on met en œuvre 1 à 4 moles de peroxyde d'hydrogène ou 1 à 1,2 mole de peroxydisulfate par mole d'acide 2-(4,4'-bis-diméthylaminobenzhydryl)-5-diméthylamino-benzoïque.